(19) Europäisches Patentamt
European Patent Office
Office européen des brevets

(11) Veröffentlichungsnummer: **0 011 596**
**B1**

(12) # EUROPÄISCHE PATENTSCHRIFT

(45) Veröffentlichungstag der Patentschrift: **30.12.81**

(51) Int. Cl.³: **C 07 D 233/22,** A 01 N 43/50

(21) Anmeldenummer: **79810112.7**

(22) Anmeldetag: **03.10.79**

(54) **Neue 2-(alpha-Phenoxy-alkyl)-imidazoline und ihre Salze, deren Herstellung und Verwendung sowie sie enthaltende Mittel zur Bekämpfung von pflanzenschädlichen und tierparasitären Milben.**

(30) Priorität: **09.10.78 CH 10469/78**

(43) Veröffentlichungstag der Anmeldung: **28.05.80 Patentblatt 80/11**

(45) Bekanntmachung des Hinweises auf die Patenterteilung: **30.12.81 Patentblatt 81/52**

(84) Benannte Vertragsstaaten: **BE CH DE FR GB IT NL**

(56) Entgegenhaltungen:
**AT-B-296 285**
**DE-A1-2 756 638**
**DE-A1-2 818 367**

(73) Patentinhaber: **CIBA-GEIGY AG, Patentabteilung Postfach, CH-4002 Basel (CH)**

(72) Erfinder: **Brechbühler, Hans U., Dr., Paracelsusstrasse 63, CH-4058 Basel (CH)**
Erfinder: **Mattern, Günter, Dr., Sichternstrasse 35, CH-4410 Liestal (CH)**
Erfinder: **Traber, Walter, Dr., Neueneichweg 12, CH-4153 Reinach (CH)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG.

# Neue 2-(alpha-Phenoxy-alkyl)-imidazoline und ihre Salze, deren Herstellung und Verwendung sowie sie enthaltende Mittel zur Bekämpfung von pflanzenschädlichen und tierparasitären Milben.

Die vorliegende Erfindung betrifft neue 2-(Phenoxy-α-alkyl)-imidazoline, Verfahren zu ihrer Herstellung und die Verwendung dieser Verbindungen als mitizide Wirkstoffe im Pflanzenschutz und zur Bekämpfung von tierparasitären Milben.

Die erfindungsgemässen 2-(Phenoxy-α-alkyl)-imidazoline haben die Formel I

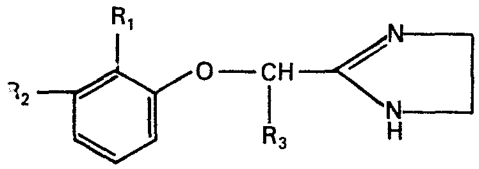

(I),

worin

$R_1$ und $R_2$ unabhängig voneinander Methyl oder Chlor, oder zusammen eine $-(CH_2)_3-$ oder $-(CH_2)_4-$ Gruppe und

$R_3$ Äthyl, n-Propyl oder i-Propyl

bedeuten.

Als hochwirksam, insbesondere gegen Pflanzen schädigende Milben, haben sich solche Verbindungen der Formel I erwiesen, worin

$R_1$ und $R_2$ unabhängig voneinander Methyl oder Chlor, und

$R_3$ Äthyl oder n-Propyl

bedeuten.

Zu den erfindungsgemässen Verbindungen bzw. den erfindungsgemäss zu verwendenden Wirkstoffen zählen auch die Additionssalze von Verbindungen der Formel I mit anorganischen und organischen Säuren. Bevorzugt sind die Additionssalze von anorganischen Säuren, z.B. der Chlorwasserstoffsäure, Bromwasserstoffsäure, Jodwasserstoffsäure, Schwefelsäure, Salpetersäure usw. Von den Additionssalzen organischer Säuren sind speziell die Exalate, Fumarate, Tartrate und Citrate zu erwähnen. Die Herstellung der genannten Additionssalze aus den Verbindungen der Formel I kann in üblicher Weise erfolgen.

Aus den deutschen Offenlegungsschriften 1695555, 1795843 und 1935479 sind verschiedenartig substituierte Phenoxyalkylimidazoline als pharmazeutische Wirkstoffe bekannt. Ferner werden in den deutschen Offenlegungsschriften 2756638 und 2756639 substituierte Phenoxymethylimidazoline als Mittel zur Bekämpfung von Arthropoden vorgeschlagen. Die dort genannten Verbindungen sind hauptsächlich zur Bekämpfung von ektoparasitären Vertretern der Ordnung Acarina, insbesondere Säugetiere befallenden Zecken, geeignet.

Es wurde nun gefunden, dass die erfindungsgemäss verwendeten Verbindungen der Formel I und deren Säureadditionssalze eine überraschend hohe und spezifische Wirksamkeit gegen Pflanzen schädigende Milben und gegen tierparasitäre Milben besitzen. So können die Verbindungen der Formel I zur Bekämpfung von phytophagen Milben der Familien Tetranychidae und Phytoptipalpidae (Spinnmilben), Tarsonemidae (Weichhautmilben) und Eriophyidae (Gallmilben) eingesetzt werden. Die Verbindungen der Formel I eignen sich vor allem zur Bekämpfung der folgenden, Obst- und Gemüsekulturen befallenden Milbenspezies: Tetranychus urticae, Tetranychus cinnabarinus, Panonychus ulmi, Bryobia rubrioculus, Panonychus citri, Eriophyes pyri, Eriophyes ribis, Eriophyes vitis, Tarsomemus pallidus, Phyllocoptes vitis und Phyllocoptura oleivora. Mit Hilfe von Verbindungen der Formel I können auch tierparasitäre Milben der Familien Sarcoptidae, Psoroptidae, Dermanyssidae und Demodicidae, insbesondere Räudemilben der Spezies Sarcoptes scabiei und Notoedres cati, welche sich tief in die Epidermis der befallenden Haus- und Nutztiere bis an die Nervenenden einbohren und intensive Irritationen und Schädigungen bei den Tieren verursachen, ferner die Spezies Dermanyssus gallinae und Psoroptes ovis bekämpft werden.

Die neue Verbindungen der Formel I können nach an sich bekannten Verfahren, beispielsweise einem der folgenden, hergestellt werden.

a) Umsetzung eines Alkalimetallsalzes eines Phenols der Formel II

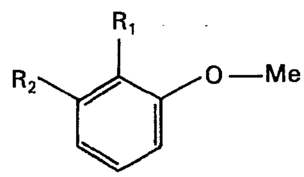

(II)

mit einem 2-(α-Halogenalkyl)-imidazolin-hydrochlorid der Formel III

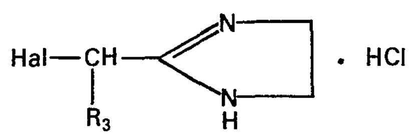

(III)

(vgl. J.Amer.Chem.Soc. 69, 1688 (1947)).

b) Umsetzung eines Imidoesters der Formel IV

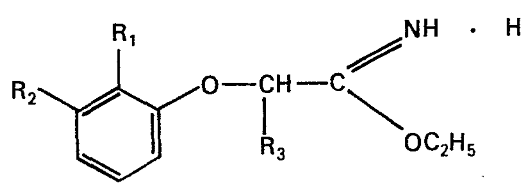

(IV)

mit Äthylendiamin (vgl. J.Amer.Chem.Soc. 69, 1688 (1947)).

c) Umsetzung eines Nitrils der Formel V,

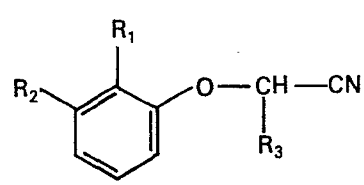

(V)

mit Äthylendiamin (vgl. J.Amer.Chem.Soc. 69, 1688 (1947)).

d) Umsetzung eines Amidinsalzes der Formel VI

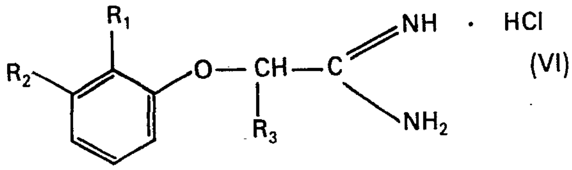

(VI)

mit Äthylendiamin (vgl. J.Amer.Chem.Soc. 69, 1688 (1947)).

e) Umsetzung eines Carbonsäureesters der Formel VII

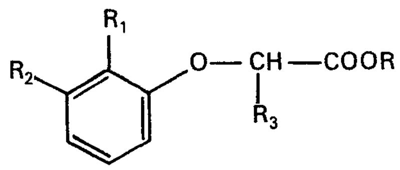

(VII)

mit Äthylendiamin (vgl. J.Amer.Chem.Soc. 69, 1688 (1947)).

f) Umsetzung eines Nitrils der Formel V mit Äthylendiamin und einem 3 bis 5 Schwefelatome enthaltenden Natriumpolysulfid (vgl. Deutsche Offenlegungsschrift Nr. 22.512.513).

g) Umsetzung eines Thioamids der Formel VIII

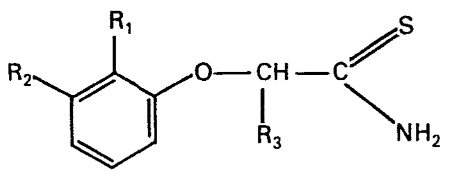

(VIII)

mit Äthylendiamin.

In den vorstehend genannten Formeln II, III, IV, V, VI, VII und VIII haben die Reste $R_1$, $R_2$ und $R_3$ die unter Formel I angegebenen Bedeutungen; Me bedeutet ein Alkalimetallatom, vorzugsweise Natrium, Hal ein Halogenatom, vorzugsweise Chlor oder Brom, und R Methyl oder Äthyl.

Die in den vorstehend aufgeführten Verfahren a) bis g) verwendeten Ausgangsstoffe sind bekannt oder können nach an sich bekannten Verfahren hergestellt werden.

So können beispielsweise Carbonsäureester der Formel VII durch Umsetzung eines in α-Stellung durch den Rest $R_3$ substituierten Halogenessigsäureniederalkylesters mit einem Phenolat der Formel II erhalten werden.

Die entsprechende Umsetzung eines in α-Stellung durch den Rest $R_3$ substituierten Halogenacetonitrils liefert Nitrile der Formel V. Die weitere Umsetzung von Nitrilen der Formel V mit Äthanol in Gegenwart von Chlorwasserstoff führt zu Hydrochloriden von Imidoestern der Formel IV, welche mit Ammoniak in Amidine der Formel VI übergeführt werden können. 2-(α-Halogenalkyl)-imidazolinhydrochloride der Formel III können schliesslich durch Umsetzung eines in α-Stellung durch den Rest $R_3$ substituierten Halogenacetimidoalkylesters mit Äthylendiamin hergestellt werden (vgl. Helv. Chimica Acta 27, 1762 (1944) und CH-PS 229.606). Nitrile der Formel V können ferner aus Carbonsäureestern der Formel VII durch Umsetzung mit Ammoniak zum entsprechenden Amid und nachfolgende Wasserabspaltung gewonnen werden. Thioamide der Formel VIII können durch Umsetzung von Nitrilen der Formel V mit Schwefelwasserstoff erhalten werden.

Die Wirkung der erfindungsgemässen Verbindungen der Formel I und der sie enthaltenden Mittel lässt sich durch Zusatz von anderen Insektiziden und/oder Akariziden wesentlich verbreitern und an gegebene Umstände anpassen.

Als Zusätze kommen z.B. folgende Wirkstoffe in Betracht:
organische Phosphorverbindungen, Nitrophenole und Derivaten, Formamidine, Harnstoffe, Carbamate und chlorierte Kohlenwasserstoffe.

Die Verbindungen der Formel I als solche oder zusammen mit geeigneten Trägern und/oder Zuschlagstoffen eingesetzt werden. Geeignete Träger und Zuschlagstoffe können fest oder flüssig sein und entsprechen den in der Formulierungstechnik üblichen Stoffen, wie z.B. natürlichen oder regenerierten Stoffen, Lösungs-, Dispergier-, Netz-, Haft-, Verdickungs- oder Bindemitteln. Zur Applikation können die Verbindungen der Formel I zu Stäubemitteln, Emulsionskonzentraten, Granulaten, Dispersionen, Sprays, zu Lösungen oder Aufschlämmungen in üblicher Formulierung, die in der Applikationstechnik zum Allgemeinwissen gehören, verarbeitet werden. Ferner sind «cattle dips», d.h. Viehbäder, und «spray races», d.h. Sprühgänge, in denen wässrige Zubereitungen verwendet werden, zu erwähnen. Diese Zubereitungsformen sind insbesondere zur Bekämpfung tierparasitärer Schädlinge geeignet.

Die Herstellung der erfindungsgemässen Mittel erfolgt in an sich bekannter Weise durch inniges Vermischen und/oder Vermahlen von Wirkstoffen der Formel I mit den geeigneten Trägerstoffen, gegebenenfalls unter Zusatz von gegenüber den Wirkstoffen inerten Dispergier- und Lösungsmitteln. Die Wirkstoffe können in den folgenden Aufarbeitungsformen vorliegen und angewendet werden:

Feste Aufarbeitungsformen:
Stäubemittel, Streumittel, Granulate (Umhüllungsgranulate, Imprägnierungsgranulate und Homogengranulate);

Flüssige Aufarbeitungsformen:
a) in Wasser dispergierbare Wirkstoffkonzentrate: Spritzpulver (wettable powders), Pasten, Emulsionen;
b) Lösungen.

Der Gehalt an Wirkstoff in den oben beschriebenen Mitteln liegt zwischen 0,1 bis 95%.

Die Wirkstoffe der Formel I können beispielsweise wie folgt formuliert werden:

Stäubemittel: Zur Herstellung eines a) 5%igen und b) 2%igen Stäubemittels werden die folgenden Stoffe verwendet:

a) 5 Teile Wirkstoff,
95 Teile Talkum;

b) 2 Teile Wirkstoff,
1 Teil hochdisperse Kieselsäure,
97 Teile Talkum.

Die Wirkstoffe werden mit den Trägerstoffen vermischt und vermahlen.

Granulat: Zur Herstellung eines 5%igen Granulates werden die folgenden Stoffe verwendet:

5 Teile Wirkstoff,
0,25 Teile Epichlorhydrin,
0,25 Teile Cetylpolyglykoläther,
3,50 Teile Polyäthylengylkol,
91 Teile Kaolin (Korngrösse 0,3–0,8 mm).

Die Aktivsubstanz wird mit Epichlorhydrin vermischt und mit 6 Teilen Aceton gelöst, hierauf wird Polyäthylenglykol und Cetylpolyglykoläther zugesetzt. Die so erhaltene Lösung wird auf Kaolin aufgesprüht und anschliessend das Aceton im Vakuum verdampft.

Spritzpulver: Zur Herstellung eines a) 40%igen, b) und c) 25%igen d) 10%igen Spritzpulvers werden folgende Bestandteile verwendet:

a) 40 Teile Wirkstoff,
5 Teile Ligninsulfonsäure-Natriumsalz,
1 Teil Dibutylnapthalinsulfonsäure-Natriumsalz,
54 Teile Kieselsäure;

b) 25 Teile Wirkstoff,
4,5 Teile Calcium-Ligninsulfonat,
1,9 Teile Champagne-Kreide/Hydroxyäthylcellulose-Gemisch (1:1),
1,5 Teile Natrium-dibutyl-naphthalinsulfonat,
19,5 Teile Kieselsäure,
19,5 Teile Champagne-Kreide,
28,1 Teile Kaolin;

c) 25 Teile Wirkstoff,
2,5 Teile Isooctylphenoxy-polyoxyäthylen-äthanol,
1,7 Teile Champagne-Kreide/Hydroxyäthylcellulose-Gemisch (1:1),
8,3 Teile Natrimaluminiumsilikat,
16,5 Teile Kieselgur,
46 Teile Kaolin;

d) 10 Teile Wirkstoff,
3 Teile Gemisch der Natriumsalze von gesättigten Fettalkoholsulfaten,
5 Teile Naphthalinsulfonsäure/Formaldehyd-Kondensat,
82 Teile Kaolin.

Die Wirkstoffe werden in geeigneten Mischern mit den Zuschlagstoffen innig vermischt und auf entsprechenden Mühlen und Walzen vermahlen. Man erhält Spritzpulver, die sich mit Wasser zu Suspensionen jeder gewünschten Konzentration verdünnen lassen.

Emulgierbare Konzentrate: Zur Herstellung eines a) 10%igen b) 25%igen und c) 50%igen emulgierbaren Konzentrates werden folgende Stoffe verwendet:

a) 10 Teile Wirkstoff
3,4 Teile epoxydiertes Pflanzenöl,
3,4 Teile eines Kombinationsemulgators, bestehend aus Fettalkoholpolyglykoläther und Alkylaralkyl-sulfonat-Calcium-Salz,
40 Teile Dimethylformamid,
43,2 Teile Xylol;

b) 25 Teile Wirkstoff,
2,5 Teile epoxydiertes Pflanzenöl,
10 Teile eines Alkylarylsulfonat/Fettalkohol-polyglykoläther-Gemisches,
5 Teile Dimethylformamid,
57,5 Teile Xylol.

c) 50 Teile Wirkstoff,
4,2 Teile Tributylphenol-Polyglykoläther,
5,8 Teil Calcium-Dodecylbenzolsulfonat,
20 Teile Cyclohexanon
20 Teile Xylol.

Aus solchen Konzentrationen können durch Verdünnen mit Wasser Emulsionen jeder gewünschten Konzentration hergestellt werden.

Sprühmittel: Zur Herstellung eines a) 5%igen und b) 95%igen Sprühmittels werden die folgenden Bestandteile verwendet:

a) 5 Teile Wirkstoff,
1 Teil Epichlorhydrin,
94 Teile Benzin (Siedegrenzen 160–190 °C),

b) 95 Teile Wirkstoff,
5 Teile Epichlorhydrin.

Beispiel 1 (Verfahren e)

2-[α-(2,3-Dimethyl)-phenoxy-butyl]-imidazolin

23,6 g 2-(2,3-Dimethyl)-phenoxy-valeriansäuremethylester und 36 g wasserfreies Äthylendiamin werden in 60 ml absolutem Methanol gelöst und 20 Std. am Rückfluss erhitzt. Nach dem Erkalten des Reaktionsgemisches wird das Methanol am Vakuum abgezogen und das rohe Produkt während 15 Std. bei 15 Torr und 200 °C gerührt. Das noch nicht ganz erstarrte Rohprodukt wird destilliert. Es wird 2-[α-(2,3-Dimethyl)-phenoxy-butyl]-imidazolin vom Sdp.118–126 °C/0,1 Torr erhalten. Beim Abkühlen auf Raumtemperatur wird die Fraktion fest, die bei 82–84 °C schmilzt.

Herstellung der Ausgangsverbindung:

42,7 g 2,3-Dimethylphenol, 68,3 g 2-Bromvaleriansäuremethylester (Sdp. 77–80 °C/17 Torr), 70 g wasserfreies und fein gemahlenes Kaliumcarbonat und 5 g Kaliumjodid werden in 500 ml Methylisobutylketon während 30 Std. gekocht. Nach Erkalten der Suspension wird filtriert, das Filtrat eingeengt, in Äther aufgenommen, zweimal mit verdünnter Natronlauge und anschliessend mit Wasser gewaschen, über Natriumfulfat

getrocknet und nach dem Filtrieren zur Trockne eingedampft. 60 g dunkles Oel werden destilliert. Die Fraktion bei 102–104 °C/0,4 Torr liefert 2-(2,3-Dimethyl)-phenoxyvaleriansäuremethylester.

Beispiel 2

2-[α-(2,3-Dimethyl)-phenoxy-butyl]-imidazolin-hydrochlorid

Nach Lösen von 4 g 2-[α-(2,3-Dimethyl)-phenoxy-butyl]-imidazolin in 30 ml Methylenchlorid wird in die erhaltene Lösung unter Kühlung bis zur Sättigung HCl-Gas eingeleitet. Dann wird das Lösungsmittel abgezogen, unter Rühren 100 ml Äther zum Rückstand gegeben und durch Reiben mit dem Glasstab das Produkt zur Kristallisation gebracht. Nach Filtrieren und Trocknen wird reines 2-[α-(2,3-Dimethyl)-phenoxy-butyl]-imidazolin-hydrochlorid vom Smp. 137–139 °C erhalten.

Beispiel 3 (Verfahren e)

2-[α-(2,3-Dimethyl)-phenoxy-propyl]-imidazolin

13,3 g 2-(2,3-Dimethyl)-phenoxy-buttersäuremethylester und 21,6 g wasserfreies Äthylendiamin werden in 50 ml abs. Methanol gelöst und 6 Std. am Rückfluss erhitzt. Nach dem Erkalten wird das Methanol am Vakuum abgesaugt und das rohe Produkt in Form eines braunen, alsbald erstarrenden Oeles 12 Std. bei 15 Torr und 200 °C gerührt. Beim Abkühlen auf Raumtemperatur wird das Reaktionsgemisch fest. Nach dem Umkristallieren aus Hexan erhält man 2-[α-(2,3-Dimethyl)-phenoxy-propyl]-imidazolin vom Smp. 90–91 °C.

Herstellung der Ausgangsverbindung:

Analog der in Beispiel 1 beschriebenen Arbeitsweise.

Analog der in den vorstehenden Beispielen beschriebenen Arbeitsweisen werden folgende Verbindungen der Formel I hergestellt:

| $R_1$ | $R_2$ | $R_3$ | Säureadditionssalz | physikalische Daten |
|---|---|---|---|---|
| $CH_3$ | $CH_3$ | $C_2H_5$ | | Smp. 90–91 °C |
| Cl | Cl | $C_2H_5$ | | Smp. 112–114 °C |
| Cl | Cl | $C_2H_5$ | HCl | Smp. 163–164 °C |
| $CH_3$ | $CH_3$ | i-$C_3H_7$ | | Smp. 100–102 °C |
| $CH_3$ | Cl | $C_2H_5$ | | Smp. 119–120 °C |
| $CH_3$ | Cl | $C_2H_5$ | HCl | |
| $CH_3$ | $CH_3$ | $C_2H_5$ | HCl | Smp. 184–186 °C |
| $CH_3$ | $CH_3$ | $C_2H_5$ | HOOC-COOH | Smp. 110–118 °C |
| | $-(CH_2)_3-$ | $C_2H_5$ | | Smp. 80–81 °C |
| $CH_3$ | $CH_3$ | n-$C_3H_7$ | | Smp. 82–84 °C |
| $CH_3$ | $CH_3$ | n-$C_3H_7$ | HCl | Smp. 137–139 °C |
| | $-(CH_2)_4-$ | $C_2H_5$ | | Smp. 93–94 °C |
| Cl | $CH_3$ | $C_2H_5$ | | |
| Cl | $CH_3$ | n-$C_3H_7$ | | |
| Cl | $CH_3$ | i-$C_3H_7$ | | |
| $CH_3$ | Cl | n-$C_3H_7$ | | |
| Cl | Cl | n-$C_3H_7$ | | Smp. 144–145 °C |
| $CH_3$ | Cl | i-$C_3H_7$ | | |
| Cl | Cl | i-$C_3H_7$ | | |
| | $-(CH_2)_3-$ | n-$C_3H_7$ | | |
| | $-(CH_2)_4$ | n-$C_3H_7$ | | |
| | $-(CH_2)_3-$ | i-$C_3H_7$ | | |
| | $-(CH_2)_4-$ | i-$C_3H_7$ | | |

Beispiel 4

Wirkung gegen Spinnmilben

Phaseolus vulgaris (Buschbohnen) wurden 16 Stunden vor dem Test mit infestierten Blattstükken aus einer Massenzucht von Tetranychus urticae belegt. Zum Zeitpunkt der Wirkstoff-Applikation befinden sich dann sowohl Eier wie auch sämtliche bewegliche Stadien in grösserer Menge auf den Pflanzen. Auf einer Drehtelleranlage wurden die mit den Milben infizierten Pflanzen mit etwa 100 ml einer wässrigen Emulsionszubereitung, enthaltend die Wirksubstanz in einer

Konzentration von 800 ppm besprüht, so dass kein Ablaufen der Spritzemulsion eintrat. Anschliessend wurden die behandelten Pflanzen in einer Gewächshauskabine bei ca. 25°C aufgestellt. Die Auswertung erfolgte 7 Tage nach der Behandlung, wobei die prozentuale Abtötung der Eier, Larven und Adulten festgestellt wurde.

Verbindungen gemäss den vorstehenden Beispielen zeigten gute Wirkung in obigem Test.

Beispiel 5
Wirkung gegen tierparasitäre Milben

Von mit Dermanyssus gallinae befallenen Hühnern wurden Ansätze bestehend aus etwa 50 Milben verschiedener Stadien (Larven, Nymphen und Imagines) entnommen. Die Milbenansätze wurden jeweils in einer Verdünnungsreihe mit einer wässrigen Emulsion, Suspension bzw. Lösung des zu prüfenden Wirkstoffes benetzt. Hierzu wurden die Milbenansätze in einem Teströhrchen mit der den Wirkstoff enthaltenden flüssigen Zubereitung übergossen; die Flüssigkeit wurde anschliessend mit Hilfe eines Wattebausches aufgesogen. Die so behandelten Milben verblieben während 72 Stunden in dem Teströhrchen. Nach dieser Zeit wurde zur Auswertung die minimale, für eine 100%igen Abtötung der behandelten Milben erforderliche Wirkstoffkonzentration gegenüber unbehandelten Kontrollansätzen ermittelt.

Verbindungen gemäss den vorstehenden Beispielen zeigten gute Wirkung im obigen Test.

**Patentansprüche**

1. Verbindung der Formel I

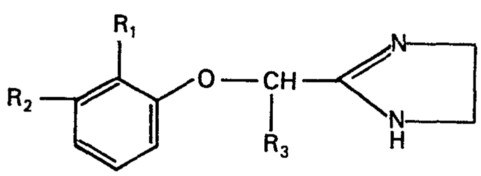

(I),

worin
$R_1$ und $R_2$ unabhängig voneinander Methyl oder Chlor, oder zusammen eine $-(CH_2)_3$ oder $-(CH_2)_4$-Gruppe und
$R_3$ Äthyl, n-Propyl oder i-Propyl
bedeuten.

2. Verbindung der Formel I nach Anspruch 1, dadurch gekennzeichnet, dass
$R_1$ und $R_2$ unabhängig voneinander Methyl oder Chlor, und
$R_3$ Äthyl oder n-Propyl
bedeuten.

3. Verbindung nach Anspruch 2 der Formel

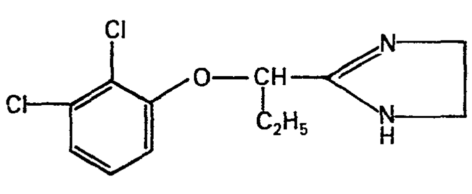

4. Verbindung nach Anspruch 2 der Formel

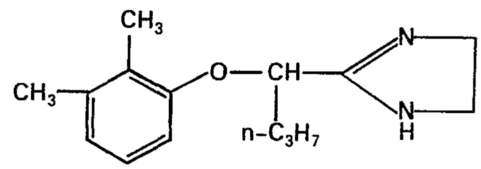

5. Verbindung nach Anspruch 2 der Formel

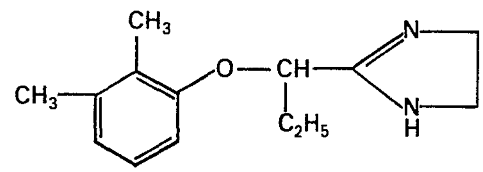

6. Verbindung nach Anspruch 2 der Formel

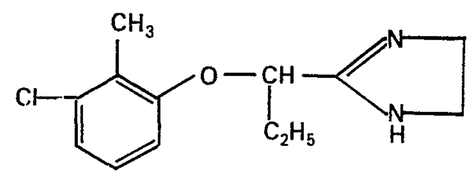

7. Verbindung nach Anspruch 2 der Formel

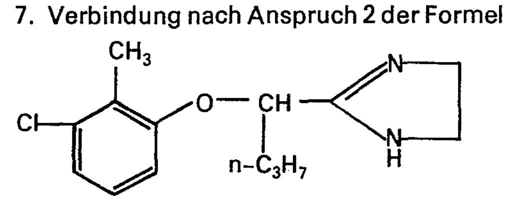

8. Verbindung nach Anspruch 2 der Formel

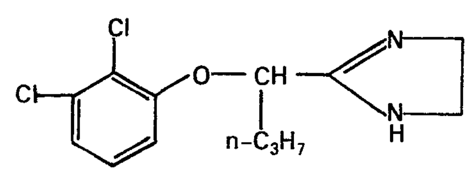

9. Verbindung nach einem der Ansprüche 1 bis 8 dadurch gekennzeichnet, dass sie in Form eines Säureadditionssalzes vorliegt.

10. Verfahren zur Herstellung einer Verbindung gemäss den Ansprüchen 1 bis 9, dadurch gekennzeichnet, dass man

a) eine Verbindung der Formel II

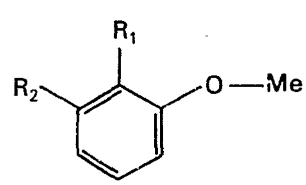

(II)

mit einer Verbindung der Formel III

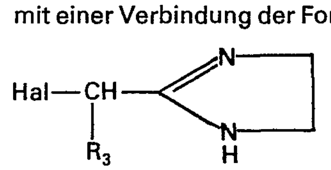

(III)

oder

b) eine Verbindung der Formel IV

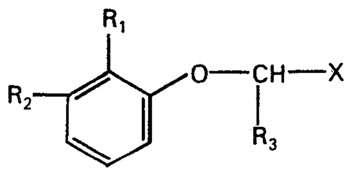

(IV)

mit Äthylendiamin umsetzt, wobei in den Formeln II bis IV die Reste $R_1$, $R_2$ und $R_3$ die in den Ansprüchen 1 und 2 angegebenen Bedeutungen haben, Me ein Alkalimetallatom, vorzugsweise Natrium, Hal Halogen, vorzugsweise Chlor oder Brom, und X einen Rest

$-C\equiv N$, 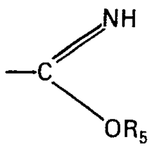 , 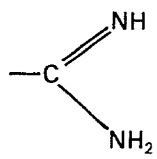 ,

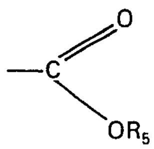 oder 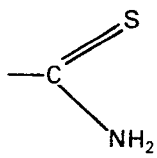

bedeuten, wobei $R_5$ für $C_1$-$C_4$-Alkyl, vorzugsweise Methyl oder Äthyl, steht, und dass man die erhaltene Verbindung der Formel I gegebenenfalls in ein Säureadditionssalz überführt.

11. Mittel zur Bekämpfung von pflanzenschädlichen Milben und tierparasitären Milben enthaltend als aktive Komponente eine Verbindung gemäss den Ansprüchen 1 bis 9 zusammen mit geeigneten Trägern und/oder anderen Zuschlagsstoffen.

12. Verwendung einer Verbindung gemäss den Ansprüchen 1 bis 9 zur Bekämpfung von pflanzenschädlichen Milben.

13. Verbindung gemäss den Ansprüchen 1 bis 9 zur Bekämpfung von tierparasitären Milben.

**Claims**

1. A compound of the formula I

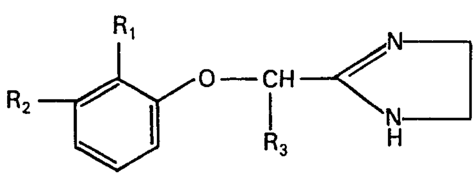

wherein $R_1$ and $R_2$, each independently of the other, are methyl or chlorine, or together are a $-(CH_2)_3$ or$-(CH_2)_4$ group, and $R_3$ is ethyl, n-propyl or isopropyl.

2. A compound of the formula I according to claim 1, wherein $R_1$ and $R_2$, each independently of the other, are methyl or chlorine, and $R_3$ is ethyl or n-propyl.

3. A compound according to claim 2 of the formula

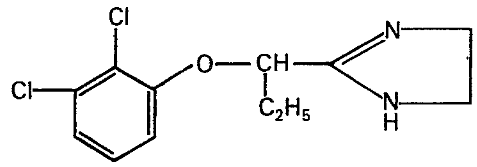

4. A compound according to claim 2 of the formula

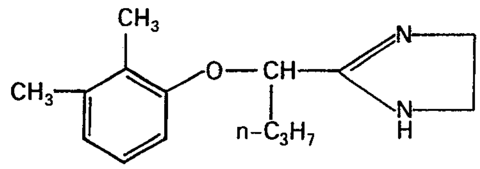

5. A compound according to claim 2 of the formula

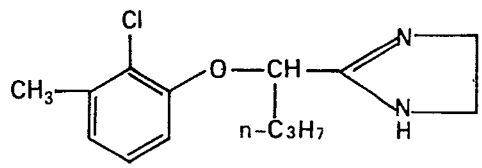

6. A compound according to claim 2 of the formula

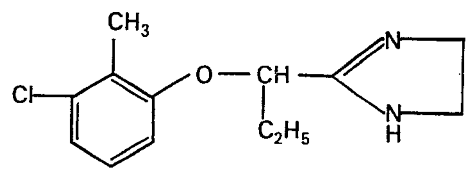

7. A compound according to claim 2 of the formula

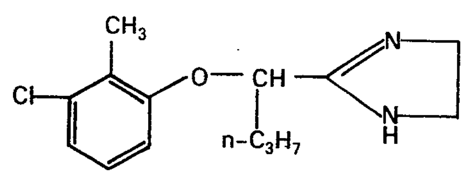

8. A compound according to claim 2 of the formula

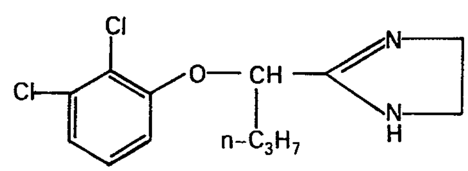

9. A compound according to any one of claims 1 to 8 which is in the form of an acid addition salt.

10. A process for the manufacture of a compound according to claims 1 to 9, which comprises reacting

a) a compound of the formula II

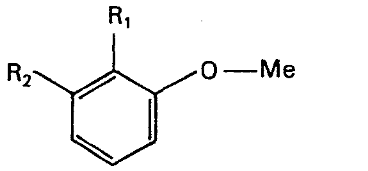

(II)

with a compound of the formula III

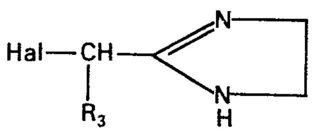

(III)

or

b) a compound of the formula IV

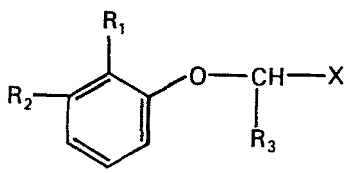

(IV)

with ethylenediamine, in which formulae II to IV the symbols $R_1$, $R_2$ and $R_3$ are as defined in claims 1 and 2, Me is an alkali metal atom, preferably sodium, Hal is halogen, preferably chlorine or bromine, and X is a radical

$-C\equiv N$, 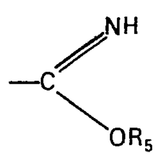 , 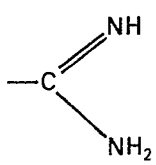 ,

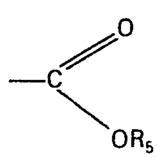 or 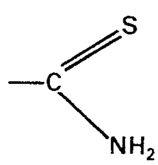

wherein $R_5$ is $C_1$-$C_4$ alkyl, preferably methyl or ethyl, and, if desired, converting the resultant compound of the formula I into an acid addition salt.

11. A composition for controlling plant-destructive mites and mites which are parasites of animals, which composition contains, as active component, a compound according to claims 1 to 9 together with suitable carriers and/or adjuvants.

12. A method of controlling plant-destructive mites and mites which are parasites of animals, which comprises the use of a compound according to claims 1 to 9.

13. A compound according to claims 1 to 9 for controlling mites which are parasites of animals.

**Revendications**

1. Composé de formule I

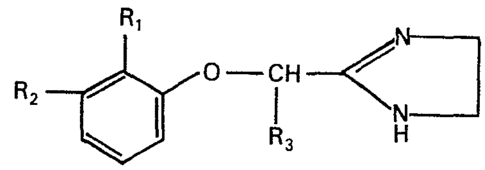

(I),

où

$R_1$ et $R_2$ représentent indépendamment l'un de l'autre un méthyle ou un chlore ou ensemble un groupe $-(CH_2)_3-$ ou $-(CH_2)_4-$ et

$R_3$ représente un éthyle, un n-propyle ou un i-propyle.

2. Composé de formule I selon la revendication 1, caractérisé en ce que

$R_1$ et $R_2$ représentent indépendamment l'un de l'autre un méthyle ou un chlore, et

$R_3$ un éthyle ou un n-propyle.

3. Composé selon la revendication 2 de formule

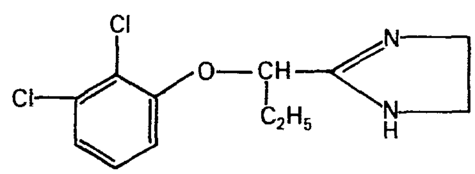

4. Composé selon la revendication 2 de formule

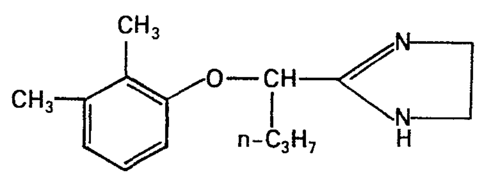

5. Composé selon la revendication 2 de formule

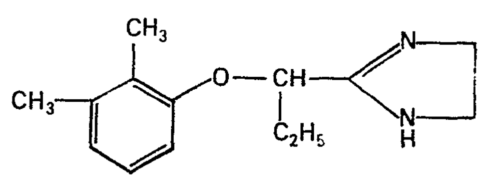

6. Composé selon la revendication 2 de formule

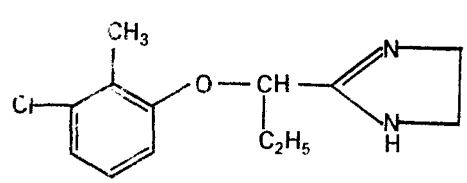

7. Composé selon la revendication 2 de formule

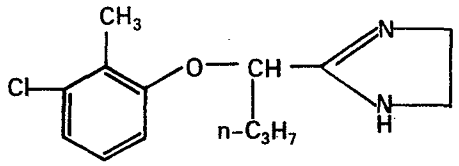

8. Composé selon la revendicaion 2 de formule

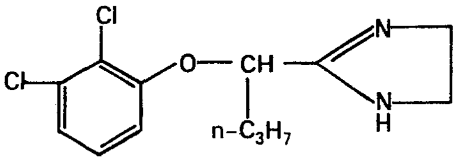

9. Composé selon l'une des revendications 1 à 8, caractérisé en ce qu'il se présente sous la forme d'un sel d'addition acide.

10. Procédé de préparation d'un composé selon les revendications 1 à 9, caractérisé en ce qu'on fait réagir

a) un composé de formule II

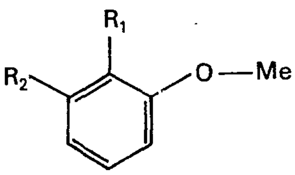 (II)

avec un composé de formule III

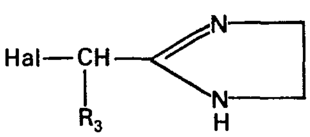 (III)

ou

b) un composé de formule IV

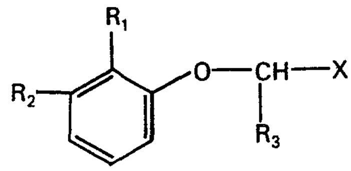 (IV)

avec l'éthylènediamine,
où dans les formules II à IV les radicaux $R_1$, $R_2$ et $R_3$ ont les significations données dans les revendications 1 et 2, Me représente un atome de métal alcalin, de préférence de sodium, Hal un halogène, de préférence le chlore ou le brome, et X un radical

$-C\equiv N$, 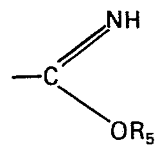 , 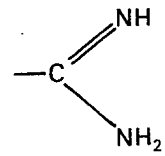 ,

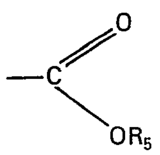 ou 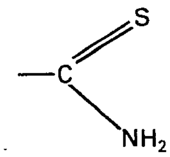

où $R_5$ représente un alcoyle en $C_1$ à $C_4$, de préférence le méthyle ou l'éthyle, et en ce qu'éventuellement on transforme le composé de formule I obtenu en un sel d'addition acide.

11. Agent de lutte contre les mites phytoparasitaires et les mites zooparasitaires contenant comme composant actif un composé selon les revendications 1 à 9 avec des supports et/ou autres additifs appropriés.

12. Application d'un composé selon les revendications 1 à 9 à la lutte contre les mites phytoparasitaires.

13. Composé selon les revendications 1 à 9 pour la lutte contre les mites zooparasitaires.